# EUROPEAN PATENT SPECIFICATION

(11) **EP 2 376 594 B1**
(45) Date of publication and mention of the grant of the patent: **06.07.2016**
(21) Application number: 09835188.5
(22) Date of filing: 03.12.2009
(51) Int. Cl.: C09K 11/06, H05B 33/14, C07D 401/10, C07D 403/10

(54) **COMPOUND FOR ORGANIC PHOTOELECTRIC DEVICE AND ORGANIC PHOTOELECTRIC DEVICE INCLUDING THE SAME**
VERBINDUNG FÜR EINE ORGANISCHE PHOTOELEKTRISCHE VORRICHTUNG SOWIE SIE ENTHALTENDE ORGANISCHE PHOTOELEKTRISCHE VORRICHTUNG
COMPOSÉ DESTINÉ À UN DISPOSITIF PHOTOÉLECTRIQUE ORGANIQUE ET DISPOSITIF PHOTOÉLECTRIQUE ORGANIQUE COMPRENANT CE COMPOSÉ

(30) Priority: 24.12.2008 KR 20080133692; 05.08.2009 KR 20090072155
(43) Date of publication of application: 19.10.2011
(73) Proprietor: CHEIL INDUSTRIES INC., Kumi-city Gyeongsangbuk-do 730-030 (KR)
(72) Inventor: KIM, Nam-Soo, Uiwang-si Gyeonggi-do 437-801 (KR); KANG, Myeong-Soon, Uiwang-si Gyeonggi-do 437-711 (KR); JUNG, Ho-Kuk, Uiwang-si Gyeonggi-do 437-711 (KR); IN, Kyu-Yeol, Uiwang-si Gyeonggi-do 437-711 (KR); KANG, Eui-Su, Uiwang-si Gyeonggi-do 437-711 (KR); LEE, Seung-Gyoung, Uiwang-si Gyeonggi-do 437-711 (KR); PARK, Young-Sung, Uiwang-si Gyeonggi-do 437-711 (KR); LEE, Hyon-Gyu, Uiwang-si Gyeonggi-do 437-711 (KR); CHAE, Mi-Young, Uiwang-si Gyeonggi-do 437-711 (KR)
(74) Representative: Michalski Hüttermann & Partner Patentanwälte mbB
(86) International application number: PCT/KR2009/007199
(87) International publication number: WO 2010/074422

(56) References cited:
- EP-A1- 1 571 193
- EP-A1- 1 718 121
- EP-A1- 1 820 801
- EP-A1- 1 930 329
- EP-A1- 2 468 731
- EP-A2- 2 383 323
- WO-A1-2005/085387
- WO-A1-2007/029696
- WO-A2-2010/024572
- JP-A- 2002 212 181
- JP-A- 2003 045 662
- US-A1- 2004 115 476
- CUI ET AL: "Diboron and triboron compounds based on linear and star-shaped conjugated ligands with 8-hydroxyquinolate functionality: impact of intermolecular interaction and boron coordination on luminescence.", THE JOURNAL OF ORGANIC CHEMISTRY, vol. 71, no. 17, 1 August 2006 (2006-08-01), pages 6485-6496, XP55027438, ISSN: 0022-3263, DOI: 10.1021/jo060841+

## Description

### Technical Field

This disclosure relates to a compound for an organic photoelectric device being capable of providing an organic photoelectric device having life-span, efficiency, electrochemical stability, and thermal stability due to excellent electron transport capability and thermal stability, and an organic photoelectric device including the same.

### Background Art

An organic photoelectric device is a device requiring a charge exchange between an electrode and an organic material by using a hole or an electron.

An organic photoelectric device may be classified as follows in accordance with its driving principles. A first organic photoelectric device is an electron device driven as follows: excitons are generated in an organic material layer by photons from an external light source; the excitons are separated to electrons and holes; and the electrons and holes are transferred to different electrodes from each other as a current source (voltage source).

A second organic photoelectric device is an electron device driven as follows: a voltage or a current is applied to at least two electrodes to inject holes and/or electrons into an organic material semiconductor positioned at an interface of the electrodes; and then the device is driven by the injected electrons and holes.

As examples, the organic photoelectric device includes an organic light emitting diode (OLED), an organic solar cell, an organic photo-conductor drum, an organic transistor, an organic memory device, etc., and it requires a hole injecting or transporting material, an electron injecting or transporting material, or a light emitting material.

An organic light emitting diode (OLED) has recently drawn attention due to an increase in demand for flat panel displays. In general, organic light emission refers to transformation of electrical energy to photo-energy.

The organic light emitting diode transforms electrical energy into light by applying current to an organic light emitting material. It has a structure in which a functional organic material layer is interposed between an anode and a cathode. The organic material layer includes multi-layer including different materials from each other, for example a hole injection layer (HIL), a hole transport layer (HTL), an emission layer, an electron transport layer (ETL), and an electron injection layer (EIL), in order to improve efficiency and stability of an organic light emitting diode.

In such an organic light emitting diode, when a voltage is applied between an anode and a cathode, holes from the anode and electrons from the cathode are injected to an organic material layer. The generated excitons generate light having certain wavelengths while shifting to a ground state.

In 1987, Eastman Kodak, Inc. firstly developed an organic light emitting diode including a low molecular aromatic diamine and aluminum complex as an emission-layer-forming material (Applied Physics Letters. 51, 913, 1987). C.W Tang et al. firstly disclosed a practicable device as an organic light emitting diode in 1987 (Applied Physics Letters, 51 12, 913-915, 1987).

According to the reference, the organic layer has a structure in which a thin film (hole transport layer (HTL)) of a diamine derivative and a thin film of tris(8-hydroxy-quinolate)aluminum (Alq₃) are stacked.

Recently, it is has become known that a phosphorescent light emitting material can be used for a light emitting material of an organic light emitting diode in addition to the fluorescent light emitting material (D. F. O'Brien et al., Applied Physics Letters, 74 3, 442-444, 1999; M. A. Baldo et al., Applied Physics letters, 75 1, 4-6, 1999). Such a phosphorescent material emits lights by transiting the electrons from a ground state to an exited state, non-radiance transiting of a singlet exciton to a triplet exciton through intersystem crossing, and transiting a triplet exciton to a ground state to emit light.

As described above, in an organic light emitting diode, an organic material layer includes a light emitting material and a charge transport material, for example a hole injection material, a hole transport material, an electron transport material, an electron injection material, and so on.

The light emitting material is classified as blue, green, and red light emitting materials according to emitted colors, and yellow and orange light emitting materials to emit colors approaching natural colors.

When one material is used as a light emitting material, a maximum light emitting wavelength is shifted to a long wavelength or color purity decreases because of interactions between molecules, or device efficiency decreases because of a light emitting quenching effect. Therefore, a host/dopant system is included as a light emitting material in order to improve color purity and increase luminous efficiency and stability through energy transfer.

In order to implement the above excellent performance of an organic light emitting diode, a material constituting an organic material layer, for example a hole injection material, a hole transport material, a light emitting material, an electron transport material, an electron injection material, and a light emitting material such as a host and/or a dopant should be stable and have good efficiency. However, development of an organic material layer forming material for an organic light emitting diode has not been satisfactory up to now and thus there is a need for a novel material. This material development is also required for other organic photoelectric devices.

Compounds for electroluminescent devices are *inter alia* disclosed in the EP 1 718 121, the EP 1 930 392, the EP 1 571 193 and the WO 2005/085387

### Disclosure of Invention

### Technical Problem

One aspect of this disclosure provides a compound for an organic photoelectric device that can act as a light emitting, or electron injection and/or transport material, and also as a light emitting host along with an appropriate dopant.

Another aspect of this disclosure provides an organic photoelectric device including the compound, and having improved life-span, efficiency, driving voltage, electrochemical stability, and thermal stability.

### Solution to Problem

One aspect of this disclosure provides a compound for an organic photoelectric device that can act as a light emitting, or electron injection and/or transport material, and also as a light emitting host along with an appropriate dopant.

Another aspect of this disclosure provides an organic photoelectric device including the compound, and having improved life-span, efficiency, driving voltage, electrochemical stability, and thermal stability.

According to one aspect of this disclosure, a compound for an organic photoelectric device represented by the following Chemical Formula 1 is provided.

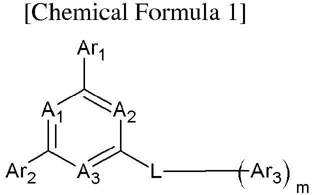

In the above Chemical Formula 1,
A₁ to A₃ are the same or different, and independently carbon or nitrogen, provided that two of A₁ to A₃ are nitrogen and one is carbon,
Ar₁ and Ar₂ are the same or different, and independently selected from the group consisting of naphthyl, anthracenyl, phenanthrenyl, pyrenyl, perylenyl, chrysenyl,
Ar₃ is unsubstituted C2 to C30 heteroaryl,
L is unsubstituted phenylene, naphthalene, or anthracene, and
m is an integer ranging from 1 to 3.

According to another aspect of this disclosure, an organic photoelectric device is provided that includes an anode, a cathode, and an organic thin layer disposed between the anode and cathode. The organic thin layer includes the material for the organic photoelectric device.

According to a further aspect of this disclosure, a display device including the organic photoelectric device is provided.

Hereinafter, other aspects of the present invention will be described in detail.

### Advantageous Effects of Invention

The compound for an organic photoelectric device includes an aryl and a heteroaryl linked to a pyrimidine core. The pyrimidine core structure including the heteroaryl reinforces electron transport capability in improvement of efficiency and a driving voltage. The pyrimidine core structure including the aryl has excellent electrochemical and thermal stability, improving life-span characteristics of an organic photoelectric device. The compound for an organic photoelectric device can act as a light emitting, or electron injection and/or transport material, and also as a light emitting host along with an appropriate dopant in an organic photoelectric device. Therefore, it provides an organic photoelectric device having improved life-span characteristics due to excellent electrochemical and thermal stability, and high luminous efficiency at a low driving voltage.

### Brief Description of Drawings

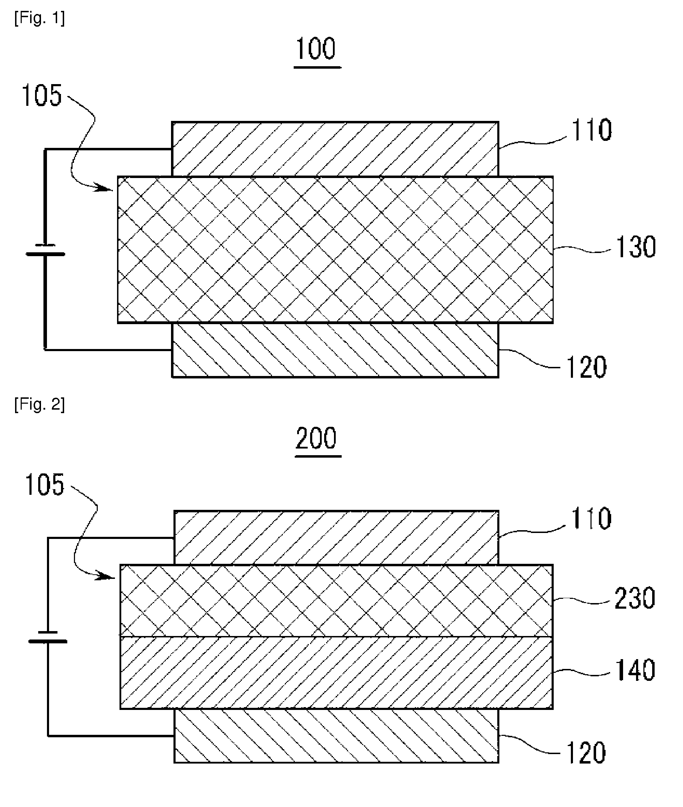

FIGS. 1 to 5 are cross-sectional views showing organic light emitting diodes including compounds according to various embodiments of the present invention.

### <Description of Reference Numerals Indicating Primary Elements in the Drawings>

100 : organic photoelectric device 110 : cathode
120 : anode 105 : organic thin layer
130 : emission layer 140 : hole transport layer (HTL)
150 : electron transport layer (ETL) 160 : electron injection layer (EIL)
170 : hole injection layer (HIL) 230 : emission layer + electron transport layer (ETL)

### Best Mode for Carrying out the Invention

Exemplary embodiments of the present invention will hereinafter be described in detail. However, these embodiments are only exemplary, and the present invention is not limited thereto.

As used herein, when specific definition is not otherwise provided, the term "hetero" refers to one including 1 to 3, including N, O, S, P, or Si, and remaining carbons in one ring.

The compound for an organic photoelectric device according to one embodiment includes a pyrimidine core, and an aryl and a heteroaryl linked to the core. The compound for an organic photoelectric device includes various substituents linked to the pyrimidine core to have various energy band gaps, and therefore can satisfy properties required in an electron injection layer (EIL), an electron transport layer, and an emission layer. The compound having various energies due to the various substituents reinforces electron transport capability resulting in improvement of efficiency and driving voltage, and life-span characteristics due to excellent electrochemical and thermal stability during operation.

According to one embodiment, the compound for an organic photoelectric device represented by the following Chemical Formula 1 is provided.

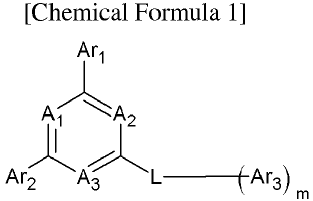

In the above Chemical Formula 1,
A₁ to A₃ are the same or different, and independently carbon or nitrogen, provided that two of A₁ to A₃ are nitrogen and one is carbon,
Ar₁ and Ar₂ are the same or different, and independently selected from the group consisting of naphthyl, anthracenyl, phenanthrenyl, pyrenyl, perylenyl, chrysenyl,
Ar₃ is unsubstituted C2 to C30 heteroaryl,
L is unsubstituted phenylene, naphthalene, or anthracene, and
m is an integer ranging from 1 to 3.
when A₁ to A₃ are carbon, they refer to CR where R is hydrogen or C1 to C15 alkyl.

The pyrimidine core including the aryl has excellent thermal stability or oxidation resistance, resulting in improvement of life-span characteristics of an organic photoelectric device.

When L is phenylene, intermolecular interaction increases to improve thermal stability, and π-conjugation length is controlled to adjust light emitting in a visible region. Thereby, it may be applicable to an emission layer of an organic photoelectric device.

The compound of the above Chemical Formula 1 according to one embodiment includes a pyrimidine core and substituents of Ar₁ to Ar₃. The pyrimidine has relatively high thermal stability or oxidation resistance, and substituent includes various substituents at 2, 4, and 6 positions due to reactivity difference.

Examples of the compound represented by the above Formula 1 include the following Chemical Formulae 3 to 5, 9, 10, 12 to 14, 18, 19, 21 to 23, 27, 28, 30 to 32, 36, 37, 39, 40, 42, 43, 45, 46, 48, and 49, but are not limited thereto (the remaining Chemical Formulae represent comparative examples).

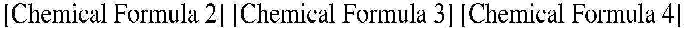

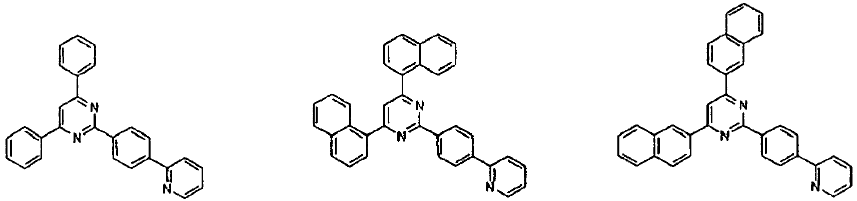

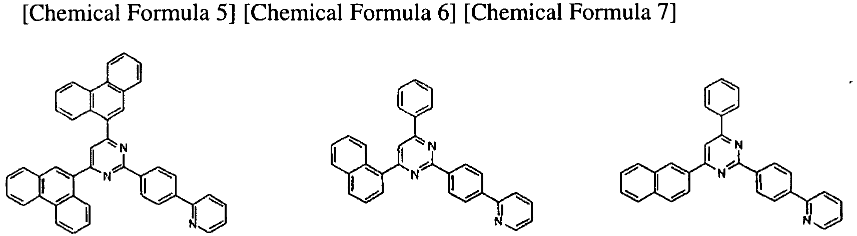

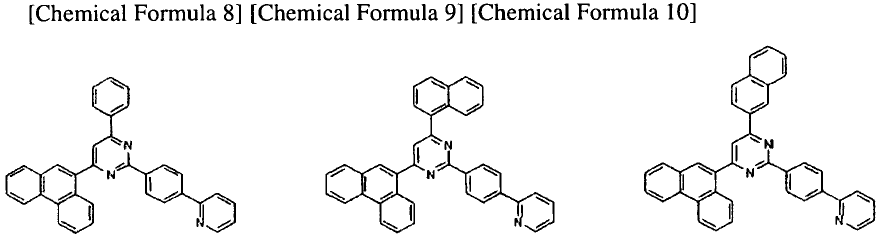

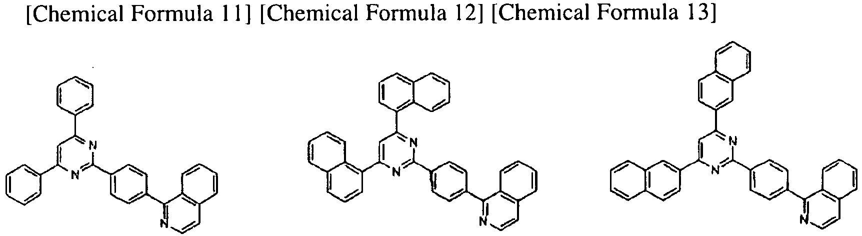

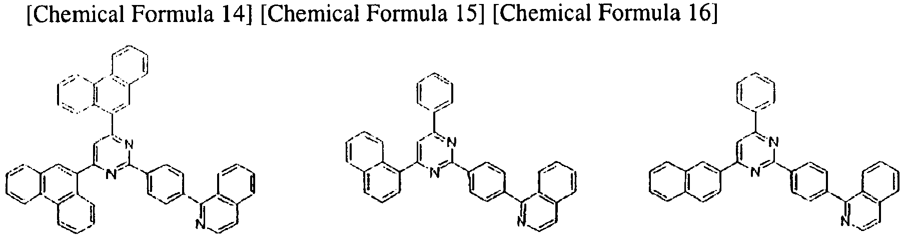

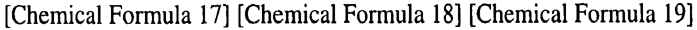

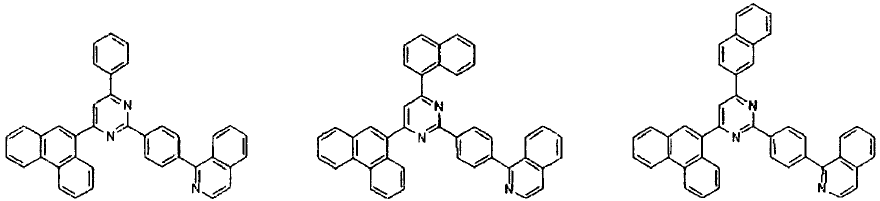

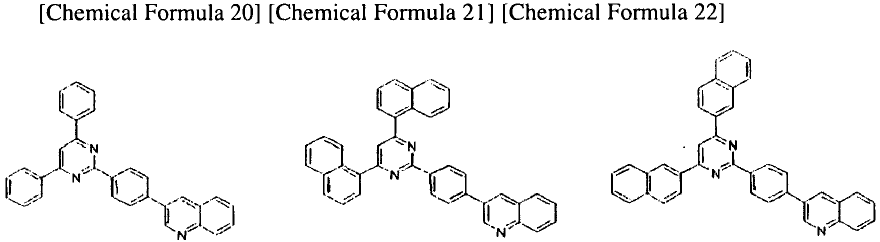

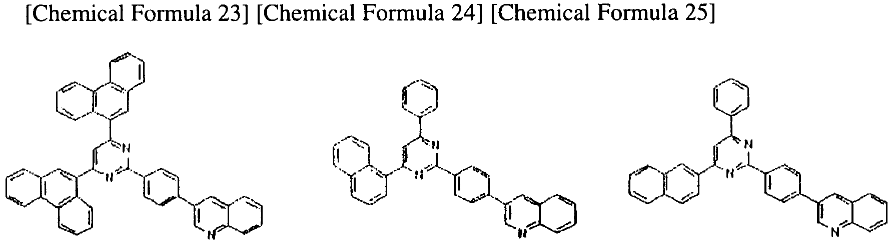

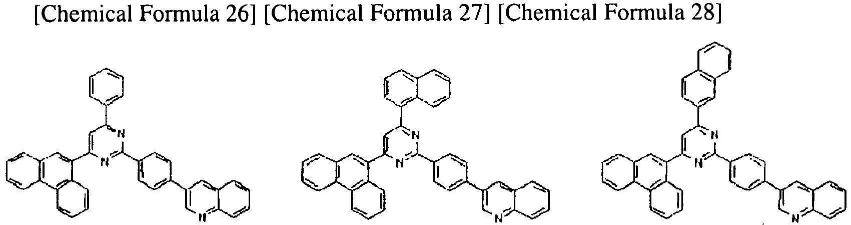

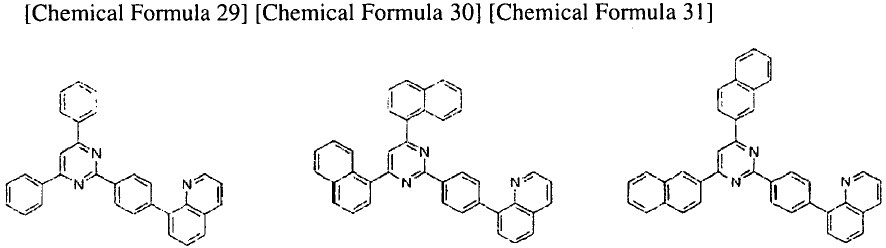

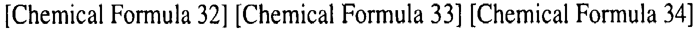

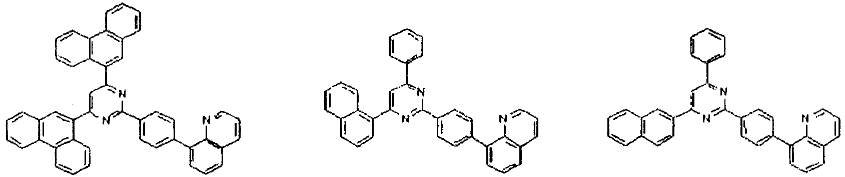

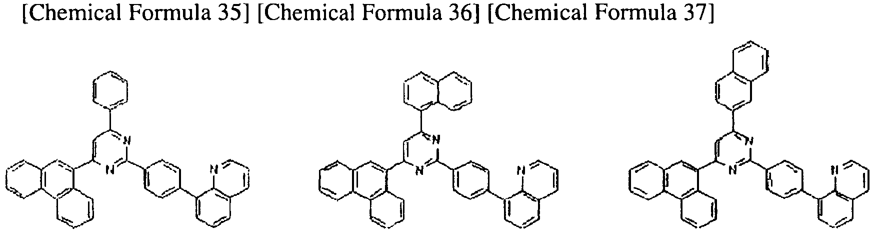

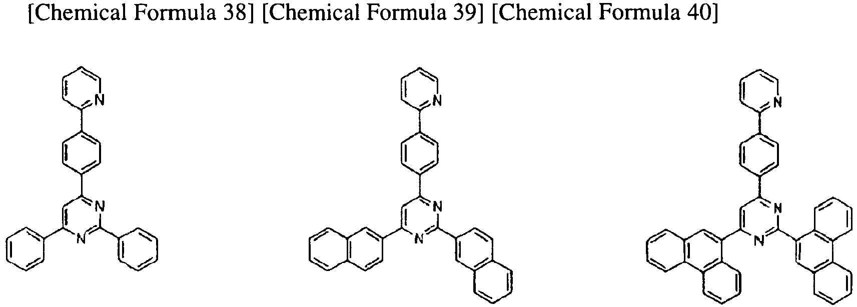

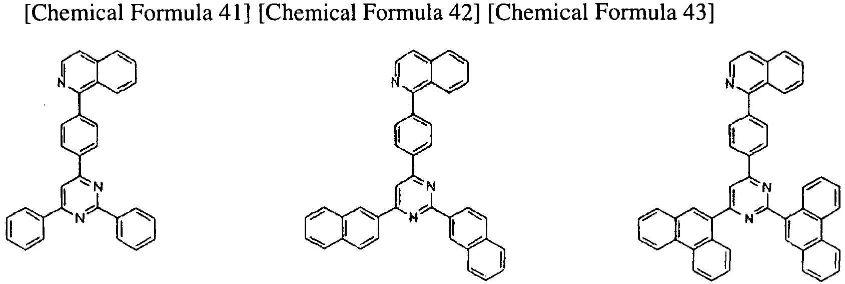

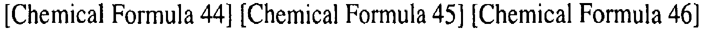

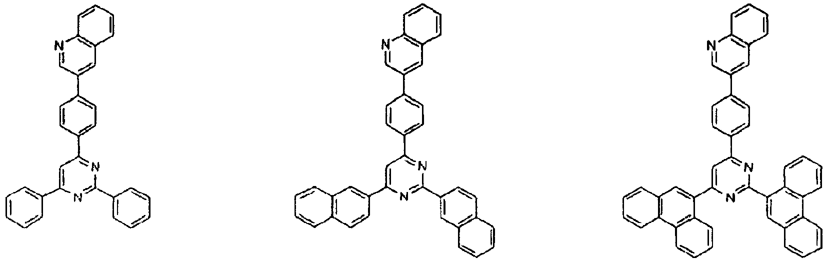

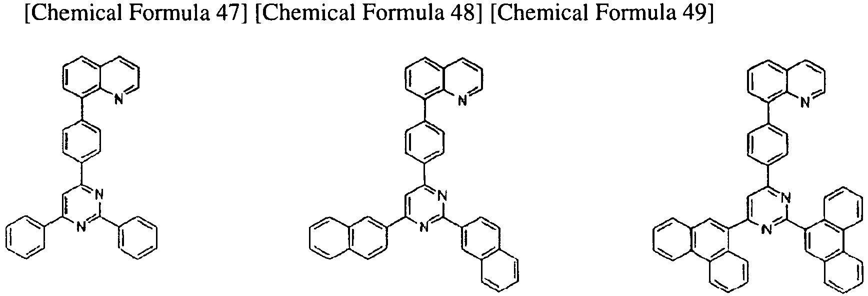

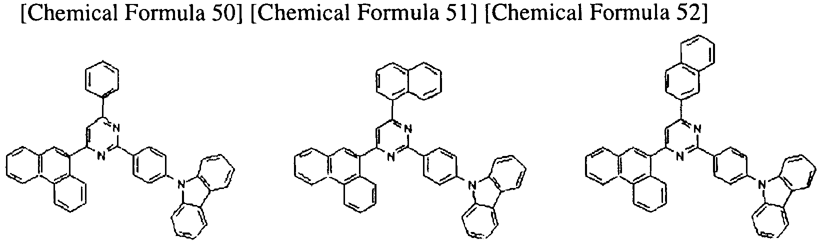

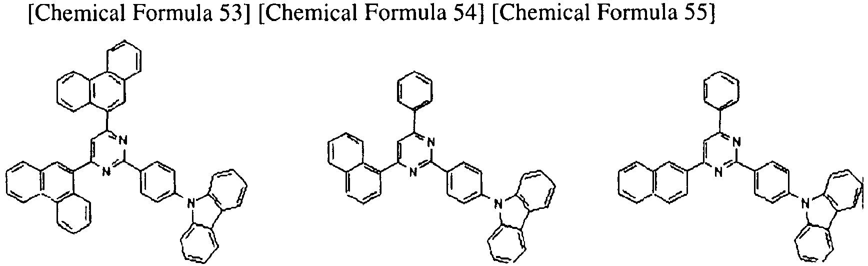

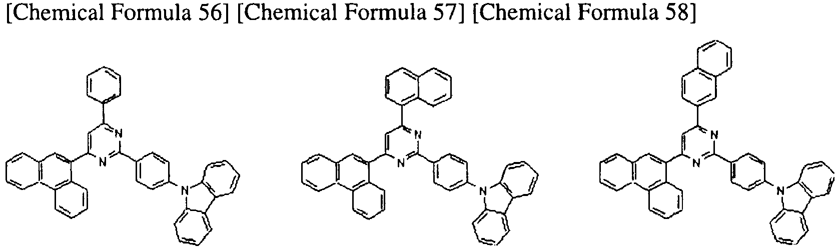

The compound for an organic photoelectric device including the above compounds can play a role of light emitting, or electron injection and/or transport, and also as a light emitting host with an appropriate dopant. The compound for an organic photo-electric device may be used for phosphorescent or fluorescent host materials, blue light emitting dopant materials, or electron transport materials.

The compound for an organic photoelectric device according to one embodiment can improve life-span and electrochemical and thermal stability, and decrease the driving voltage for improving the life-span and efficiency characteristics of an organic photo-electric device when included in an organic thin layer.

According to another embodiment of the present invention, an organic photoelectric device including the compound for the organic photoelectric device is provided. The organic photoelectric device includes an organic light emitting diode, an organic solar cell, an organic transistor, an organic photo-conductor drum, an organic memory device, and the like. As for an organic solar cell, the compound is included in an electrode or an electrode buffer layer and can thereby improve quantum efficiency. As for an organic transistor, it can be used as an electrode material in a gate, a source-drain electrode, and the like.

Hereinafter, an organic light emitting diode is illustrated in more detail.

According to another embodiment of the present invention, an organic light emitting diode including an anode and a cathode, and at least one organic thin layer disposed between the anode and cathode is provided. The organic thin layer includes the compound for an organic photoelectric device.

The organic thin layer is selected from the group consisting of an emission layer; and a hole transport layer (HTL), a hole injection layer (HIL), an electron transport layer (ETL), an electron injection layer (EIL), a hole blocking layer, and combinations thereof. At least one layer includes the compound for an organic photoelectric device according to one embodiment. In particular, an electron transport layer (ETL) or electron injection layer (EIL) includes the compound for an organic photoelectric device according to one embodiment. When the compound for an organic photoelectric device is included in an emission layer, the compound for an organic photoelectric device acts as a phosphorescent or fluorescent host, or particularly, a fluorescent blue dopant material.

FIGS. 1 to 5 are cross-sectional views showing an organic light emitting diode including the material for an organic photoelectric device according to one embodiment of the present invention.

Referring to FIGS. 1 to 5, organic light emitting diodes 100, 200, 300, 400, and 500 according to one embodiment includes at least one organic thin layer 105 interposed between an anode 120 and a cathode 110.

The anode 120 includes an anode material laving a large work function to help hole injection into an organic thin layer. The anode material includes a metal such as nickel, platinum, vanadium, chromium, copper, zinc, and gold, or alloys thereof; a metal oxide such as zinc oxide, indium oxide, indium tin oxide (ITO), and indium zinc oxide (IZO); a combined metal and oxide such as ZnO:Al or SnO₂:Sb; or a conductive polymer such as poly(3-methylthiophene), poly[3,4-(ethylene-1,2-dioxy)thiophene] (PEDT), polypyrrole, and polyaniline, but is not limited thereto. It is preferable to include a transparent electrode including ITO (indium tin oxide) as an anode.

The cathode 110 includes a cathode material having a small work function to help electron injection into an organic thin layer. The cathode material includes a metal such as magnesium, calcium, sodium, potassium, titanium, indium, yttrium, lithium, gadolinium, aluminum, silver, tin, and lead, or alloys thereof; or a multi-layered material such as LiF/Al, Liq/Al, LiO₂/Al, LiF/Ca, LiF/Al, and BaF₂/Ca, but is not limited thereto. It is preferable to include a metal electrode including aluminum as a cathode.

Referring to FIG. 1, the organic photoelectric device 100 includes an organic thin layer 105 including only an emission layer 130.

Referring to FIG. 2, a double-layered organic photoelectric device 200 includes an organic thin layer 105 including an emission layer 230 including an electron transport layer (ETL), and a hole transport layer (HTL) 140. The emission layer 130 also functions as an electron transport layer (ETL), and the hole transport layer (HTL) 140 layer has an excellent binding property with a transparent electrode such as ITO or an excellent hole transporting property.

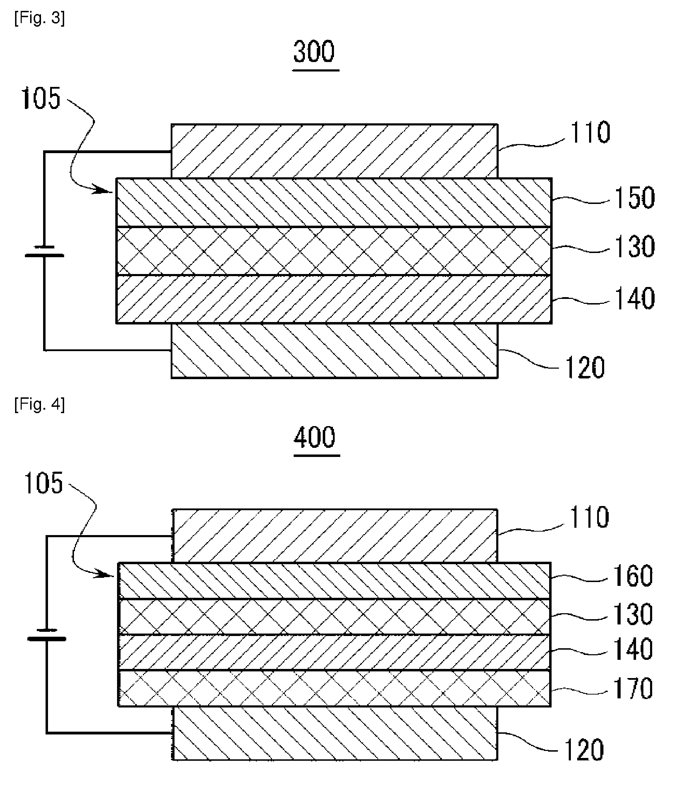

Referring to FIG. 3, a three-layered organic photoelectric device 300 includes an organic thin layer 105 including an electron transport layer (ETL) 150, an emission layer 130, and a hole transport layer (HTL) 140. The emission layer 130 is independently installed, and layers having an excellent electron transporting property or an excellent hole transporting property are separately stacked.

As shown in FIG. 4, a four-layered organic photoelectric device 400 includes an organic thin layer 105 including an electron injection layer (EIL) 160, an emission layer 130, a hole transport layer (HTL) 140, and a hole injection layer (HIL) 170 for binding with the cathode of ITO.

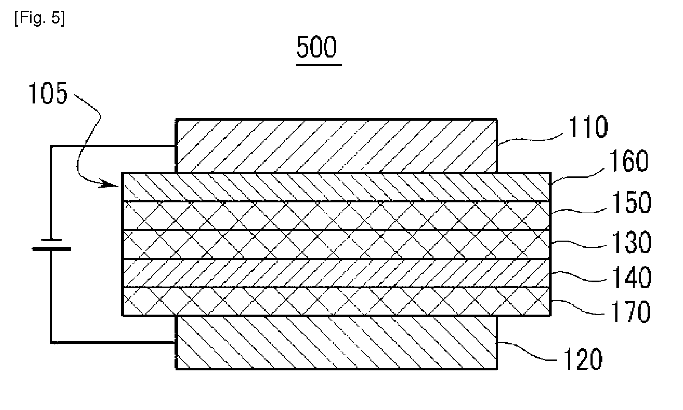

As shown in FIG. 5, a five layered organic photoelectric device 500 includes an organic thin layer 105 including an electron transport layer (ETL) 150, an emission layer 130, a hole transport layer (HTL) 140, and a hole injection layer (HIL) 170, and further includes an electron injection layer (EIL) 160 to achieve a low voltage.

In FIG. 1 to FIG. 5, the organic thin layer 105 including at least one selected from the group consisting of an electron transport layer (ETL) 150, an electron injection layer (EIL) 160, an emission layer 130 and 230, a hole transport layer (HTL) 140, a hole injection layer (HIL) 170, and combinations thereof includes a material for an organic photoelectric device. The material for the organic photoelectric device may be used for an electron transport layer (ETL) 150 including the electron transport layer (ETL) 150 or electron injection layer (EIL) 160. When it is used for the electron transport layer (ETL), it is possible to provide an organic photoelectric device having a more simple structure because it does not require an additional hole blocking layer (not shown).

Furthermore, when the compound for an organic photoelectric device is included in the emission layer 130 and 230, the material for the organic photoelectric device may be included as a phosphorescent or fluorescent host or a fluorescent blue dopant.

The organic light emitting diode may be fabricated by forming an anode on a substrate; forming an organic thin layer in accordance with a dry coating method such as an evaporation, a sputtering, a plasma plating, and an ion plating or a wet coating method such as spin coating, dipping, and flow coating; and providing a cathode thereon.

Another embodiment of the present invention provides a display device including the organic photoelectric device according to the above embodiment.

### Mode for the Invention

Hereinafter, the embodiments are illustrated in more detail with reference to examples. However, the following are exemplary embodiments and are not limiting.

### (Preparing a compound for an organic photoelectric device)

### Example 1: Synthesis of Chemical Formula 4 compound

For the specific example of the compound for an organic photoelectric device, the compound of the above Chemical Formula 4 as synthesized in accordance with the following Reaction Scheme 1 in two steps.

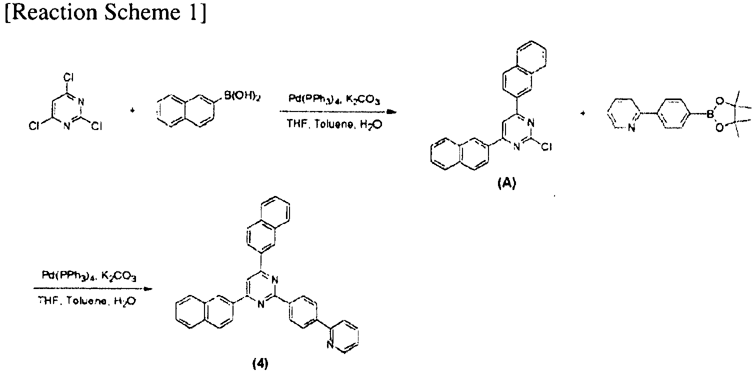

### First step; Synthesis of intermediate product A

20.0 g (109 mmol) of 2,4,6-trichloropyrimidine, 37.5 g (218 mmol) of 2-naphthylboronic, and 6.3 g (5.5 mmol) of tetrakis-(triphenylphosphine)palladium were suspended in a mixed solvent of 600 ml of tetrahydrofuran and 400 ml of toluene to provide a suspension. The suspension was added to a solution in which 60.3 g (436 mmol) of potassium carbonate was dissolved in 400 ml of water, and the obtained mixture was heated and refluxed for 9 hours. After separating the reaction fluid into two layers, an organic layer was washed with a saturated sodium chloride aqueous solution and dried with anhydrous sodium sulfate.

The organic solvent was distillated and removed under reduced pressure, and then the residue was recrystallized with toluene. The obtained crystal was separated by filtration and washed with toluene to obtain 29.5 g (yield: 73.8 %) of intermediate product (A).

### Second step: Synthesis of Chemical Formula 4 compound

5.0 g (14 mmol) of the intermediate product (A) obtained in the first step, 4.2 g (15 mmol) of 2- (4-(4,4,5,5-tetramethyl-1,3,2-dioxaborolan-2-yl)phenyl)pyridineand 0.39 g (0.3 mmol) of tetrakis-(triphenylphosphine)palladium were suspended in a mixed solvent of 150 ml of tetrahydrofuran and 100 ml of toluene to provide a suspension. The suspension was added to a solution in which 3.8 g (27 mmol) of potassium carbonate was dissolved in 100 ml of water. The obtained mixture was heated and refluxed for 12 hours. After separating the reaction fluid into two layers, an organic layer was washed with a saturated sodium chloride aqueous solution and dried with anhydrous sodium sulfate.

The organic solvent was distillated and removed under reduced pressure, and then the residue was recrystallized with toluene. The obtained crystal was separated by filtration and washed with toluene to obtain 5.3 g (yield: 80.5 %) of compound of Chemical Formula 4.

MS[M+1] 486.

### Example 2: Synthesis of Chemical Formula 10 compound

For the specific example of the compound for an organic photoelectric device, compound of the above Chemical Formula 10 was synthesized in accordance with the following Reaction Scheme 2 in three steps.

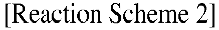

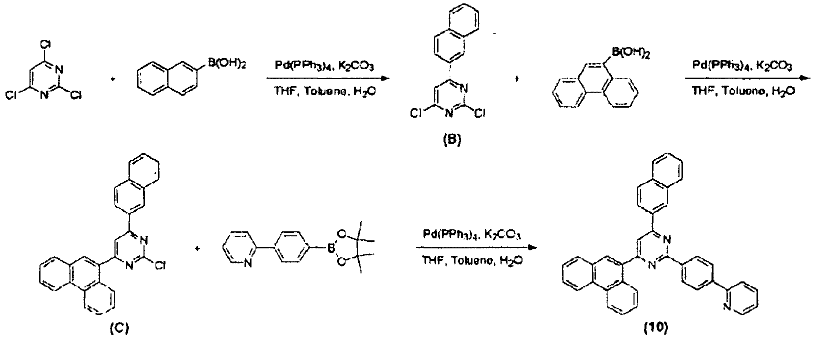

### First step; Synthesis of intermediate product (B)

20.0 g (109 mmol) of 2,4,6-trichloropyrimidine, 18.8 g (109 mmol) of 2-naphthylboronic acid, and 3.2 g (2.8 mmol) of tetrakis-(triphenylphosphine)palladium were suspended in a mixed solvent of 600 ml of tetrahydrofuran and 400 ml of toluene to provide a suspension. The suspension was added to a solution in which 30.2 g (218 mmol) of potassium carbonate was dissolved in 400 ml of water, and the obtained mixture was heated and refluxed for 9 hours. After separating the reaction fluid into two layers, an organic layer thereof was washed with a saturated sodium chloride aqueous solution and dried with anhydrous sodium sulfate.

The organic solvent was distillated and removed under reduced pressure, and then the residue was recrystallized with toluene. The obtained crystal was separated by filtration and washed with toluene to obtain 20.1 g (yield: 67.1 %) of intermediate product (B).

### Second step; Synthesis of intermediate product (C)

20.0 g (73 mmol) of the intermediate product (B) obtained in the first step, 16.2 g (73 mmol) of 9-phenanthreneboronic acid, and 2.1 g (1.8 mmol) of tetrakis-(triphenylphosphine)palladium were suspended in a mixed solvent of 600 ml of tetrahydrofuran and 400 ml of toluene to provide a suspension. The suspension was added to a solution in which 20.2 g (146 mmol) of potassium carbonate was dissolved in 400 ml of water, and the obtained mixture was heated and refluxed for 9 hours. After separating the reaction fluid into two layers, an organic layer thereof was washed with a saturated sodium chloride aqueous solution and dried with anhydrous sodium sulfate.

The organic solvent was distillated and removed under reduced pressure, and then the residue was recrystallized with toluene. The obtained crystal was separated by filtration and washed with toluene to obtain 19.4 g (yield: 63.9 %) of intermediate product (C).

### Third step; Synthesis of Chemical Formula 10 compound

5.8 g (14 mmol) of the intermediate product (C) obtained in the second step, 4.2 g (15 mmol) of 2-(4- (4,4,5,5-tetramethyl-1,3,2-dioxaborolan-2-yl)phenyl)pyridine and 0.39 g (0.3 mmol) of tetrakis-(triphenylphosphine)palladium were suspended in a mixed solvent of 170 ml of tetrahydrofuran and 115 ml of toluene to provide a suspension. The suspension was added to a solution in which 3.8 g (27 mmol) of potassium carbonate was dissolved in 115 ml of water. The obtained mixture was heated and refluxed for 12 hours. After separating the reaction fluid into two layers, an organic layer thereof was washed with a saturated sodium chloride aqueous solution and dried with anhydrous sodium sulfate.

The organic solvent was distillated and removed under reduced pressure, and then the residue was recrystallized with toluene. The extracted crystal was separated by filtration and washed with toluene to obtain 5.1 g (yield: 67.5 %) of compound of Chemical Formula 10.

MS[M+1] 536.

### Example 3: Synthesis of Chemical Formula 13 compound

For the specific example of the material for organic photoelectric device, the compound of the above Chemical Formula 13 was synthesized in accordance with the following Reaction Scheme 3.

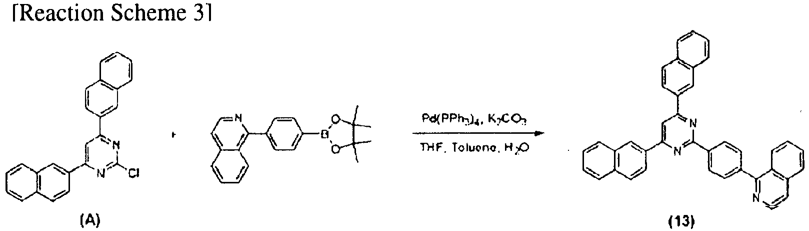

5.0 g (14 mmol) of the intermediate product (A) obtained in the first step of Example 1, 5.0 g (15 mmol) of 1-(4-(4,4,5,5-tetramethyl-1,3,2-dioxaborolan-2-yl)phenyl)isoquinoline, and 0.39 g (0.3 mmol) of tetrakis-(triphenylphosphine)palladium were suspended in a mixed solvent of 150 ml of tetrahydrofuran and 100 ml of toluene to provide a suspension. The suspension was added to a solution in which 3.8 g (27 mmol) of potassium carbonate was dissolved in 100 ml of water. The obtained mixture was heated and refluxed for 12 hours. After separating the reaction fluid into two layers, an organic layer thereof was washed with a saturated sodium chloride aqueous solution and dried with anhydrous sodium sulfate.

The organic solvent was distillated and removed under reduced pressure, and then the residue was recrystallized with toluene. The extracted crystal was separated by filtration and washed with toluene to obtain 6.2 g (yield: 82.2 %) of compound of Chemical Formula 13.

MS[M+1] 536.

### Example 4: Synthesis of Chemical Formula 22 compound

For a specific example of the material for organic photoelectric device, the compound of the above Chemical Formula 22 was synthesized in accordance with the following Reaction Scheme 4.

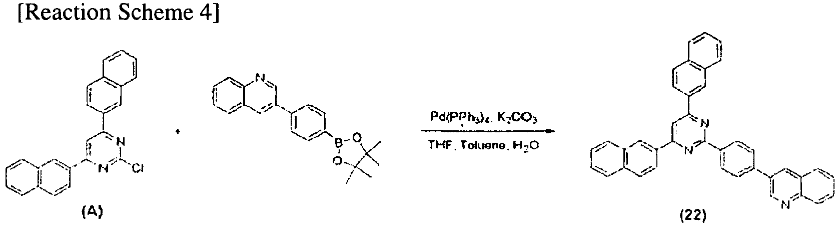

5.0 g (14 mmol) of the intermediate product (A) obtained in the first step of Example 1, 5.0 g (15 mmol) of 3-(4-(4,4,5,5-tetramethyl-1,3,2-dioxaborolan-2-yl)phenyl)quinoline and 0.39 g (0.3 mmol) of tetrakis-(triphenylphosphine)palladium were suspended in a mixed solvent of 150 ml of tetrahydrofuran and 100 ml of toluene to provide a suspension. The suspension was added to a solution in which 3.8 g (27 mmol) of potassium carbonate was dissolved in 100 ml of water. The obtained mixture was heated and refluxed for 12 hours. After separating the reaction fluid into two layers, an organic layer thereof was washed with a saturated sodium chloride aqueous solution and dried with anhydrous sodium sulfate.

The organic solvent was distillated and removed under reduced pressure, and then the residue was recrystallized with toluene. The extracted crystal was separated by filtration and washed with toluene to obtain 4.8 g (yield: 63.4 %) of compound of Chemical Formula 22.

MS[M+1] 536.

### Example 5: Synthesis of Chemical Formula 28 compound

For the specific example of the material for organic photoelectric device, the compound of the above Chemical Formula 28 was synthesized in accordance with the following Reaction Scheme 5.

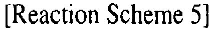

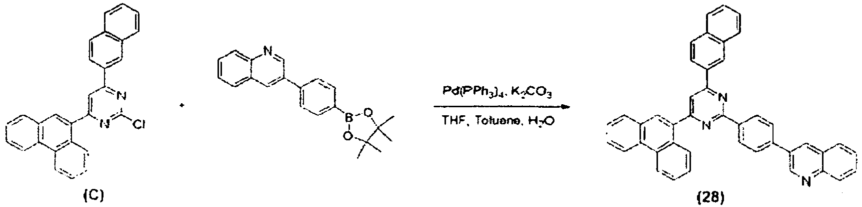

5.8 g (14 mmol) of the intermediate product (C) obtained in the first step of Example 2, 5.0 g (15 mmol) of 3-(4-(4,4,5,5-tetramethyl-1,3,2-dioxaborolan-2-yl)phenyl)quinoline, and 0.39 g (0.3 mmol) of tetrakis-(triphenylphosphine)palladium were suspended in a mixed solvent of 170 ml of tetrahydrofuran and 115 ml of toluene to provide a suspension. The suspension was added to a solution in which 3.8 g (27 mmol) of potassium carbonate was dissolved in 115 ml of water. The obtained mixture was heated and refluxed for 12 hours. After separating the reaction fluid into two layers, an organic layer thereof was washed with a saturated sodium chloride aqueous solution and dried with anhydrous sodium sulfate.

The organic solvent was distillated and removed under reduced pressure, and then the residue was recrystallized with toluene. The extracted crystal was separated by filtration and washed with toluene to obtain 5.1 g (yield: 61.9 %) of compound of Chemical Formula 28.

MS[M+1] 586.

### Example 6: Synthesis of Chemical Formula 31 compound

For a specific example of the material for organic photoelectric device, the compound of the above Chemical Formula 31 was synthesized in accordance with the following Reaction Scheme 6.

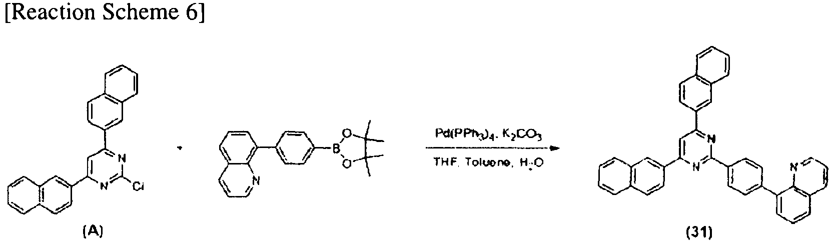

5.0 g (14 mmol) of the intermediate product (A) obtained in the first step of Example 1, 5.0 g (15 mmol) of 8-(4-(4,4,5,5-tetramethyl-1,3,2-dioxaborolan-2-yl)phenyl)quinoline, and 0.39 g (0.3 mmol) of tetrakis-(triphenylphosphine)palladium were suspended in a mixed solvent of 150 ml of tetrahydrofuran and 100 ml of toluene to provide a suspension. The suspension was added to a solution in which 3.8 g (27 mmol) of potassium carbonate was dissolved in 100 ml of water. The obtained mixture was heated and refluxed for 12 hours. After separating the reaction fluid into two layers, an organic layer thereof was washed with a saturated sodium chloride aqueous solution and dried with anhydrous sodium sulfate.

The organic solvent was distillated and removed under reduced pressure, and then the residue was recrystallized with toluene. The extracted crystal was separated by filtration and washed with toluene to obtain 6.0 g (yield: 79.5 %) of compound of Chemical Formula 31.

MS[M+1] 536.

### Example 7: Synthesis of Chemical Formula 45 compound

For a specific example of the material for organic photoelectric device, the compound of the above Chemical Formula 45 was synthesized in accordance with the following Reaction Scheme 7 in two steps.

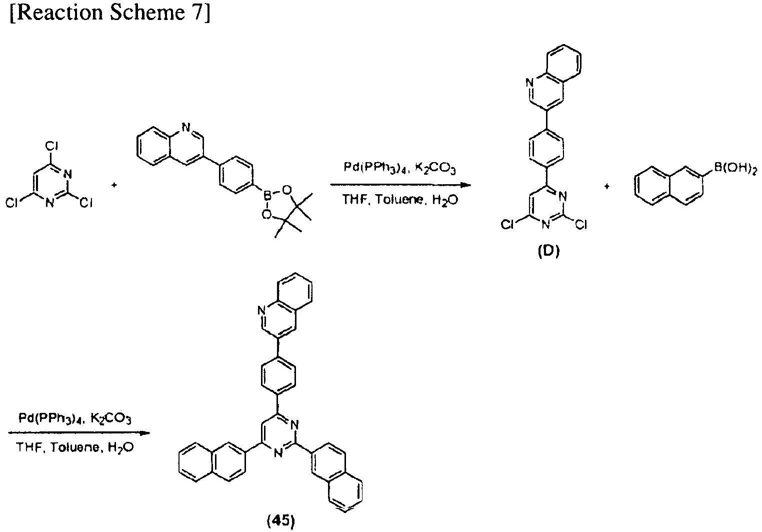

### First step; Synthesis of intermediate product (D)

20.0 g (109 mmol) of 2,4,6-trichloropyrimidine, 39.7g (120 mmol) of 3-(4-(4,4,5,5-tetramethyl-1,3,2-dioxaborolan-2-yl)phenyl)quinoline, and 3.2 g (2.7 mmol) of tetrakis-(triphenylphosphine)palladium were suspended in a mixed solvent of 600 ml of tetrahydrofuran and 400 ml of toluene to provide a suspension. The suspension was added to a solution in which 30.2 g (218 mmol) of potassium carbonate was dissolved in 400 ml of water, and the obtained mixture was heated and refluxed for 9 hours. After separating the reaction fluid into two layers, an organic layer thereof was washed with a saturated sodium chloride aqueous solution and dried with anhydrous sodium sulfate.

The organic solvent was distillated and removed under reduced pressure, and then the residue was recrystallized with toluene. The obtained crystal was separated by filtration and washed with toluene to obtain 26.3 g (yield: 68.5 %) of intermediate product (D).

### Second step; Synthesis of Chemical Formula 45 compound

4.9 g (14 mmol) of the intermediate product (D) obtained in the first step, 5.3 g (31 mmol) of 2-naphthylboronic acid, and 0.8 g (0.7 mmol) of tetrakis-(triphenylphosphine)palladium were suspended in a mixed solvent of 150 ml of tetrahydrofuran and 100 ml of toluene to provide a suspension. The suspension was added to a solution in which 7.6 g (56 mmol) of potassium carbonate was dissolved in 400 ml of water, and the obtained mixture was heated and refluxed for 12 hours. After separating the reaction fluid into two layers, an organic layer thereof was washed with a saturated sodium chloride aqueous solution and dried with anhydrous sodium sulfate.

The organic solvent was distillated and removed under reduced pressure, and then the residue was recrystallized with toluene. The obtained crystal was separated by filtration and washed with toluene to obtain 5.4 g (yield: 72.0 %) of the compound of Chemical Formula 45.

MS[M+1] 536.

### Example 8: Synthesis of Chemical Formula 46 compound

For the specific example of the material for organic photoelectric device, the compound of the above Chemical Formula 46 was synthesized in accordance with the following Reaction Scheme 8.

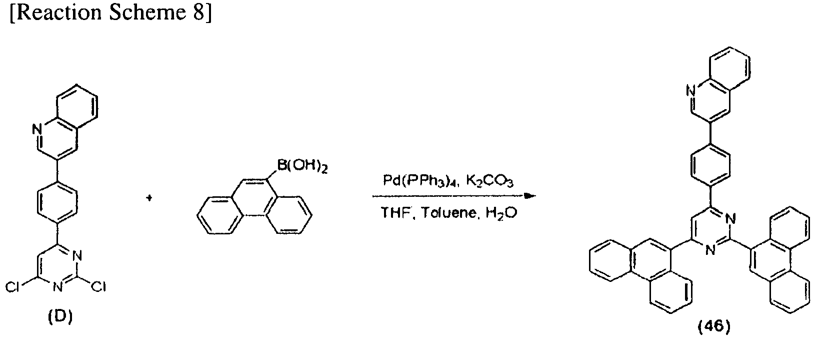

4.9 g (14 mmol) of the intermediate product (D) obtained in Example 7, 6.8 g (31 mmol) of 9-phenanthreneboronic acid, and 0.8 g (0.7 mmol) of tetrakis-(triphenylphosphine)palladium were suspended in a mixed solvent of 150 ml of tetrahydrofuran and 100 ml of toluene to provide a suspension. The suspension was added to a solution in which 7.6 g (56 mmol) of potassium carbonate was dissolved in 100 ml of water. The obtained mixture was heated and refluxed for 12 hours. After separating the reaction fluid into two layers, an organic layer thereof was washed with a saturated sodium chloride aqueous solution and dried with anhydrous sodium sulfate.

The organic solvent was distillated and removed under reduced pressure, and then the residue was recrystallized with toluene. The extracted crystal was separated by filtration and washed with toluene to obtain 6.7 g (yield: 75.3 %) of compound of Chemical Formula 46.

MS[M+1] 636.

### Example 9: Synthesis of Chemical Formula 23 compound

For the specific example of the compound for an organic photoelectric device, compound of the above Chemical Formula 23 was synthesized in accordance with the following Reaction Scheme 9 in two steps.

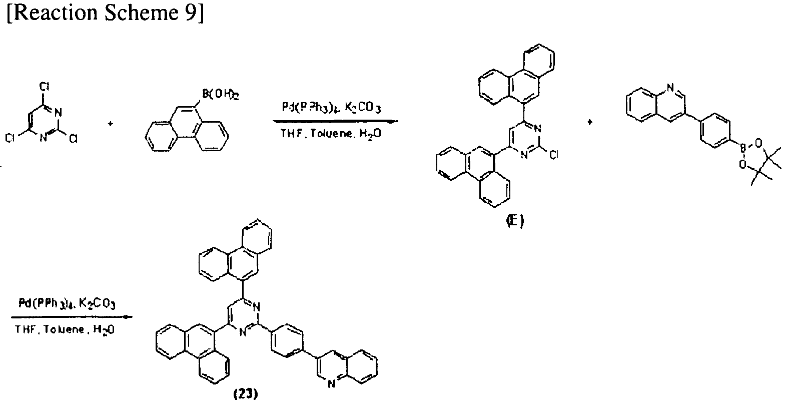

### First step; Synthesis of intermediate product (E)

20.0 g (109 mmol) of 2,4,6-trichloropyrimidine, 48.4 g (218 mmol) of 9-phenanthreneboronic acid, and 6.3 g (5.5 mmol) of tetrakis-(triphenylphosphine)palladium were suspended in a mixed solvent of 600 ml of tetrahydrofuran and 400 ml of toluene to provide a suspension. The suspension was added to a solution in which 60.3 g (436 mmol) of potassium carbonate was dissolved in 400 ml of water, and the obtained mixture was heated and refluxed for 9 hours. After separating the reaction fluid into two layers, an organic layer thereof was washed with a saturated sodium chloride aqueous solution and dried with anhydrous sodium sulfate.

The organic solvent was distillated and removed under reduced pressure, and then the residue was recrystallized with toluene. The obtained crystal was separated by filtration and washed with toluene to obtain 35.2 g (yield: 69.1 %) of intermediate product (E).

### Second step; Synthesis of Chemical Formula 23 compound

6.5 g (14 mmol) of the intermediate product (E) obtained in the first step, 5.0 g (15 mmol) of 3-(4-(4,4,5,5-tetramethyl-1,3,2-dioxaborolan-2-yl)phenyl)quinoline and 0.39 g (0.3 mmol) of tetrakis-(triphenylphosphine)palladium were suspended in a mixed solvent of 150 ml of tetrahydrofuran and 100 ml of toluene to provide a suspension. The suspension was added to a solution in which 3.8 g (27 mmol) of potassium carbonate was dissolved in 100 ml of water, and the obtained mixture was heated and refluxed for 12 hours. After separating the reaction fluid into two layers, an organic layer thereof was washed with a saturated sodium chloride aqueous solution and dried with anhydrous sodium sulfate.

The organic solvent was distillated and removed under reduced pressure, and then the residue was recrystallized with toluene. The obtained crystal was separated by filtration and washed with toluene to obtain 7.3 g (yield: 81.5 %) of the compound of Chemical Formula 23.

MS[M+1] 636.

### Example 10: Synthesis of Chemical Formula 52 compound (comparative example)

For the specific example of the compound for an organic photoelectric device, compound of the above Chemical Formula 52 was synthesized in accordance with the following Reaction Scheme 10 in three steps.

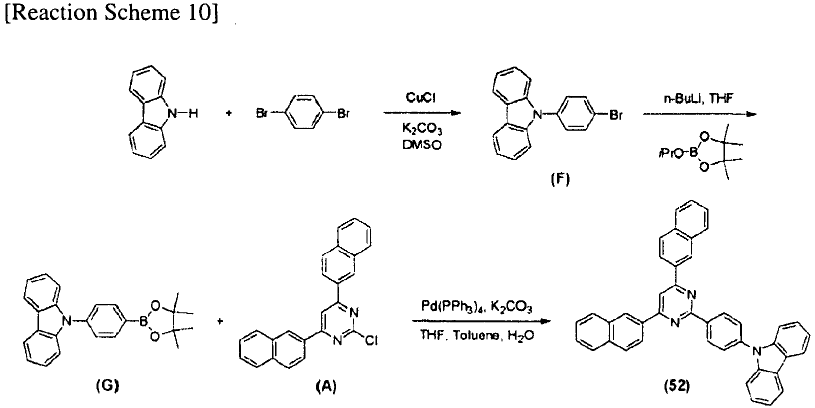

### First step; Synthesis of intermediate product (F)

50.8 g (304 mmol) of carbazole, 71.6 g (304 mmol) of 1,4-dibromobenzene, 3.76 g (38 mmol) of cuprous chloride, and 83.9 g (607 mmol) of potassium carbonate were suspended in 322 ml of dimethylsulfoxide, and refluxed for 8 hours under nitrogen atmosphere. The reaction fluid was cooled to room temperature and recrystallized using methanol.

The obtained crystal was separated by filtration and the residue was purified using a silica gel column chromatography to obtain 59.9 g (yield: 61.3 %) of the intermediate product (F).

### Second Step; Synthesis of intermediate product (G)

37.8 g (117 mmol) of the intermediate product (F) was dissolved in 378 ml of tetrahydrofuran, and (1.6 M) 100.5 ml (161 mmol) of a n-butyllithium hexane solution was added at -70 °C under argon atmosphere. The obtained solution was agitated at -70 to -40 °C for 1 hour. After the obtained reaction fluid was cooled to -70 °C, 47.9 ml (235 mmol) of isopropyltetramethyldioxaborolane was slowly added a dropwise fashion. The obtained solution was agitated at -70 °C for 1 hour, and then a temperature was increased to room temperature followed by agitating for 6 hours. 200 ml of water was added to the obtained solution followed by agitating for 20 minutes.

After separating the reaction fluid into two layers, an organic layer was washed with a saturated sodium chloride aqueous solution and dried with anhydrous sodium sulfate.

The organic solvent was distillated and removed under reduced pressure, and then the residue was recrystallized with toluene. The obtained crystal was separated by filtration and washed with toluene to obtain 28.9 g (yield: 66.7 %) of an intermediate product (G).

### Third step: Synthesis of Chemical Formula 52 compound

4.4 g (12 mmol) of the compound (A), 5.2 g (14 mmol) of the intermediate product (G), and 0.36 g (0.3 mmol) of tetrakis-(triphenylphosphine)palladium were suspended in a mixed solvent of 130 ml of tetrahydrofuran and 90 ml of toluene to provide a suspension. The suspension was added to a solution in which 3.4 g (25 mmol) of potassium carbonate was dissolved in 90 ml of water, and the obtained mixture was heated and refluxed for 12 hours. After separating the reaction fluid into two layers, an organic layer thereof was washed with a saturated sodium chloride aqueous solution and dried with anhydrous sodium sulfate.

The organic solvent was distillated and removed under reduced pressure, and then the residue was recrystallized with toluene. The obtained crystal was separated by filtration and washed with toluene to obtain 5.4 g (yield: 78.3 %) of the compound of Chemical Formula 52.

MS[M+1] 574.

### Example 11: Synthesis of Chemical Formula 5 compound

For the specific example of the compound for an organic photoelectric device, compound of the above Chemical Formula 5 was synthesized in accordance with the following Reaction Scheme 11 in three steps.

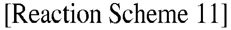

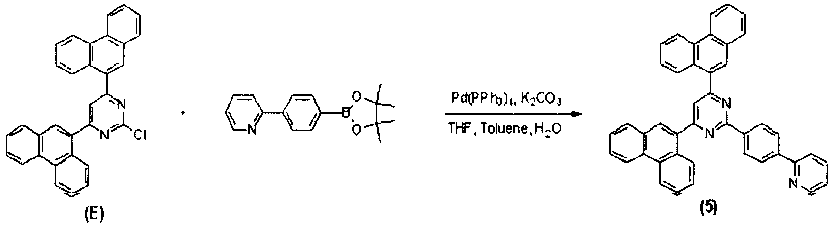

6.5 g (14 mmol) of the intermediate product (E) obtained in the first step of Example 9, 4.2 g (15 mmol) of 2-(4-(4,4,5,5-tetramethyl-1,3,2-dioxaborolan-2-yl)phenyl)pyridine, and 0.39 g (0.3 mmol) of tetrakis-(triphenylphosphine)palladium were suspended in a mixed solvent of 195 ml of tetrahydrofuran and 130 ml of toluene to provide a suspension. The suspension was added to a solution in which 3.8 g (27 mmol) of potassium carbonate was dissolved in 400 ml of water, and the obtained mixture was heated and refluxed for 12 hours. After separating the reaction fluid into two layers, an organic layer thereof was washed with a saturated sodium chloride aqueous solution and dried with anhydrous sodium sulfate.

The organic solvent was distillated and removed under reduced pressure, and then the residue was recrystallized with toluene. The obtained crystal was separated by filtration and washed with toluene to obtain 5.4 g (yield: 65.3 %) of the compound of Chemical Formula 5.

MS[M+1] 586.

### (Fabricating Organic Photoelectric Device)

### Example 12

For an anode, 1000 Åthick ITO, and for a cathode, 1000 -thick aluminum (Al) were used.

A method of fabricating an organic photoelectric device is described in more detail. A 15 W/cm² ITO glass substrate was prepared to have a size of 50 mm x 50 mm x 0.7 mm, and was cleaned in acetone, isopropyl alcohol, and pure water for 5 minutes, respectively, and then washed with UV and ozone for 30 minutes.

On the glass substrate, for a hole injection layer (HIL) N¹,N^{1'}-(biphenyl-4,4'-diyl)bis (N¹-(naphthalen-2-yl)-N⁴,N⁴-diphenylbenzene-1,4-diamine) was deposited at a thickness of 65 nm. Then, for a hole transport layer (HTL), N,N'-di (1-naphthyl)-N,N'-diphenylbenzidine (NPB) was deposited at a thickness of 40 nm.

### For an emission layer, 5 wt% N,N,N',N'-tetrakis

(3,4-dimethylphenyl)chrysene-6,12-diamine (fluorescent blue dopant) and 95 wt% of 9-(3-(naphthalen-1-yl)phenyl)-10-(naphthalen-2-yl)anthracene (fluorescent blue host) were deposited at a thickness of 25 nm.

For an electron transport layer (ETL), the compound of Chemical Formula 4 obtained in Example 1 was deposited at a thickness of 35 nm.

On the electron transport layer (ETL), for an electron injection layer (EIL), Liq was vacuum-deposited at a thickness of 0.5 nm, and Al was vacuum-deposited at a thickness of 100 nm to provide a Liq/Al electrode. The structure of the obtained organic photoelectric device is shown in FIG. 5.

### Example 13

An organic photoelectric device was fabricated in accordance with the same procedure as in Example 12, except that for an electron transport layer (ETL), the compound of Chemical Formula 22 in Example 4 was used instead of the compound of Chemical Formula 4 in Example 1.

### Example 14

An organic photoelectric device was fabricated in accordance with the same procedure as in Example 12, except that for an electron transport layer (ETL), the compound of Chemical Formula 28 in Example 5 was used instead of the compound of Chemical Formula 4 in Example 1.

### Example 15

An organic photoelectric device was fabricated in accordance with the same procedure as in Example 12, except that for an electron transport layer (ETL), the compound of Chemical Formula 45 in Example 7 was used instead of the compound of Chemical Formula 4 in Example 1.

### Example 16

An organic photoelectric device was fabricated in accordance with the same procedure as in Example 12, except that for an electron transport layer (ETL), the compound of Chemical Formula 46 in Example 8 was used instead of the compound of Chemical Formula 4 in Example 1.

### Example 17

An organic photoelectric device was fabricated in accordance with the same procedure as in Example 12, except that for an electron transport layer (ETL), the compound of Chemical Formula 23 in Example 9 was used instead of the compound of Chemical Formula 4 in Example 1.

### Example 18

An organic photoelectric device was fabricated in accordance with the same procedure as in Example 12, except that for an electron transport layer (ETL), the compound of Chemical Formula 5 in Example 11 was used instead of the compound of Chemical Formula 4 in Example 1.

### Comparative Example 1

An organic photoelectric device was fabricated in accordance with the same procedure as in Example 12, except that for an electron transport layer (ETL), the compound Alq₃ (35 nm) represented by the following Chemical Formula 59 was used instead of the compound of Chemical Formula 4 in Example 1.

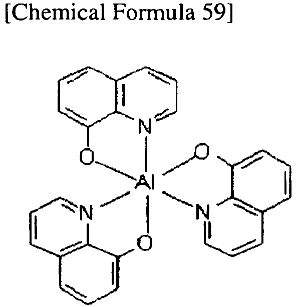

### Experimental Examples: Performance Measurement of the Organic Photoelectric Device

Each organic photoelectric device according to Examples 12 to 18 and Comparative Example 1 was measured regarding luminous efficiency according to a voltage. The measurement method is as follows.

### 1) Measurement of Current Density Change Depending on Voltage Change

The fabricated organic photoelectric devices according to Examples 12 to 18 and Comparative Example 1 were increased in voltage from 0V to 14V and measured regarding a current value in a unit device by using a current-voltage device (Keithley 2400^{®}). Then, their current densities were measured by dividing the current values by areas.

### 2) Measurement of Luminance Change Depending on Voltage Change

The organic photoelectric devices according to Examples 12 to 18 and Comparative Example 1 were increased in voltage from 0V to 14V and measured regarding luminance using a luminance meter (Minolta Cs-1000A).

### 3) Measurement of Electric Power Efficiency

Electric power efficiency was calculated from the current density and the luminance measured from the "1) Measurement of current density change depending on voltage chance" and "2) Measurement of luminance change depending on voltage change" and voltage (V). The results are shown in the following Table 1.

Table 1
[Table 1]

**[Table ]**

| | Luminance 1000 cd/m² | | | Color coordinate CIE | |
|---|---|---|---|---|---|
| | Driving voltage (V) | Current efficiency (cd/W) | Electric power efficiency (lm/W) | x | y |
| Example 12 | 5.2 | 11.10 | 6.70 | 0.15 | 0.23 |
| Example 13 | 6.0 | 9.3 | 4.85 | 0.15 | 0.23 |
| Example 14 | 6.6 | 8.5 | 4.04 | 0.15 | 0.23 |
| Example 15 | 6.4 | 7.2 | 3.55 | 0.15 | 0.23 |
| Example 16 | 7.2 | 8.6 | 3.75 | 0.15 | 0.23 |
| Example 17 | 7.7 | 8.0 | 3.27 | 0.15 | 0.23 |
| Example 18 | 5.2 | 12.0 | 7.35 | 0.15 | 0.23 |
| Comparative Example 1 | 7.3 | 6.84 | 2.94 | 0.15 | 0.23 |

Referring to Table 1, it is confirmed that the organic photoelectric devices according to Examples 1 to 5 in which the electron transport layer was formed with the material for an organic photoelectric device according to the present invention had excellent efficiency performance together with a low driving voltage, compared to the case of using the electron transport layer of Alq₃ according to Comparative Example 1. Accordingly, the organic compound according to one embodiment of the present invention had high thermal stability, a low driving voltage, and high luminous efficiency as shown in the performance result of the organic photoelectric device, so it is anticipated that it can improve the life-span of an organic photoelectric device. It is considered that the aryl substituent improves electrochemical and thermal stability, resulting in improvement of life-span of the device.

This disclosure is not limited to the embodiments illustrated with the drawings and table, but can be fabricated into various modifications andequivalent arrangements included within the spirit and scope of the appended claims by a person who is ordinarily skilled in this field. Therefore, the aforementioned embodiments should be understood to be exemplary but not limiting the present invention in any way.

## Claims

1. A compound for an organic photoelectric device represented by the following Chemical Formula 1: 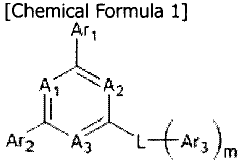 wherein in the above Chemical Formula 1,
A₁ to A₃ are the same or different, and independently carbon or nitrogen, provided that two of A₁ to A₃ are nitrogen and one is carbon,
Ar₁ and Ar₂ are the same or different, and independently selected from the group consisting of naphthyl, anthracenyl, phenanthrenyl, pyrenyl, perylenyl, chrysenyl,
Ar₃ is unsubstituted C2 to C30 heteroaryl,
L is unsubstituted phenylene, naphthalene, or anthracene, and
m is an integer ranging from 1 to 3.

2. The compound of claim 1, wherein Ar₃ comprises one selected from the group consisting of imidazole, thiazolyl, oxazolyl, oxadiazolyl, triazolyl, pyridinyl, pyridazine, quinolinyl, isoquinolinyl, acridinyl, imidazopyridinyl, imidazopyrimidinyl.

3. The compound of claim 1, wherein L is phenylene.

4. The compound of claim 1, wherein the compound comprises one represented by the following Chemical Formulae 3 to 5, 9, 10, 12 to 14, 18, 19, 21 to 23, 27, 28, 30 to 32, 36, 37, 39, 40, 42, 43, 45, 46, 48, and 49, or a mixture thereof:

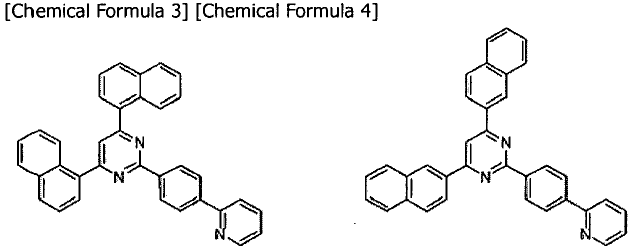

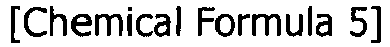

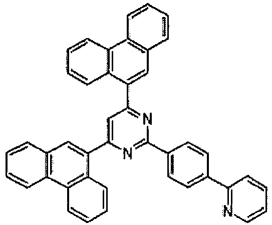

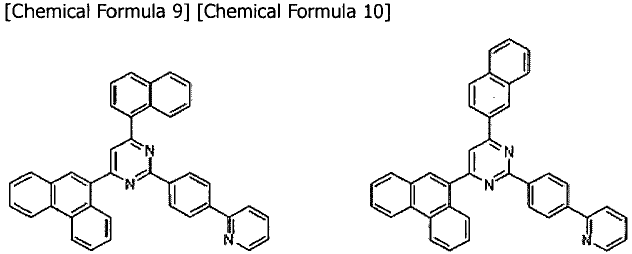

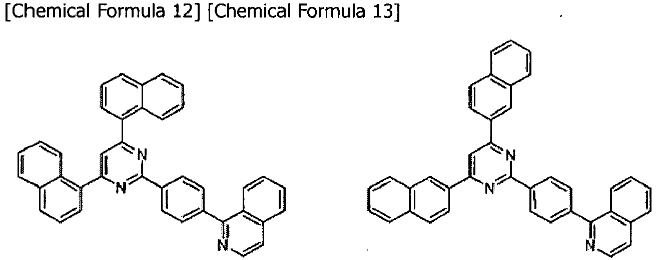

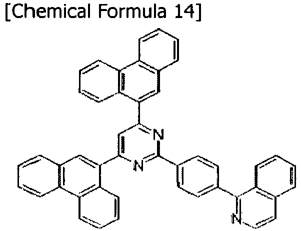

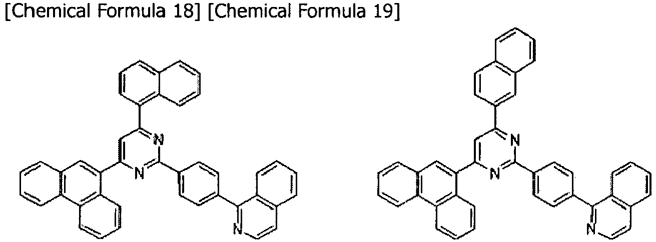

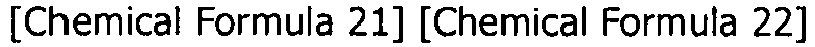

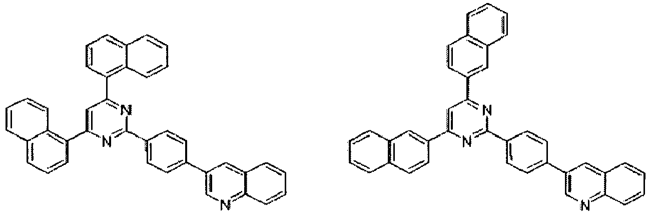

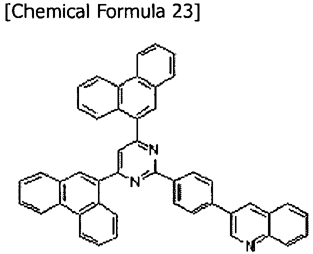

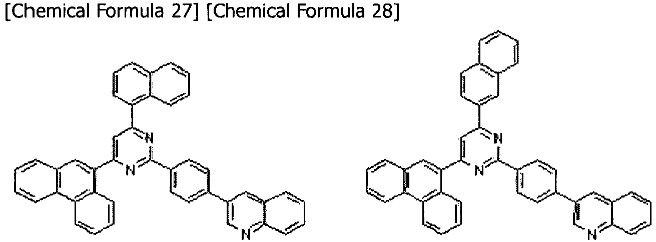

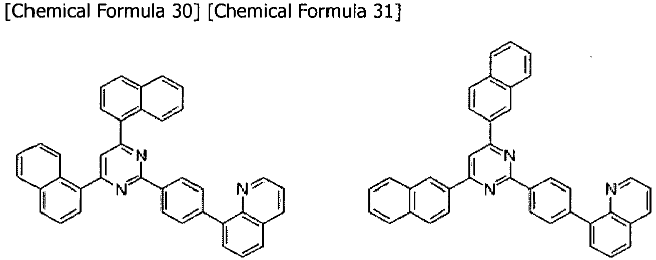

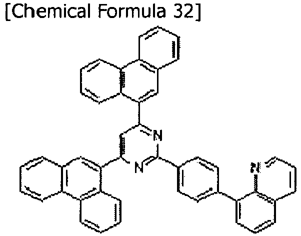

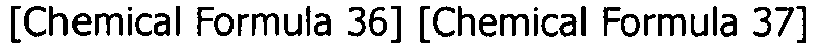

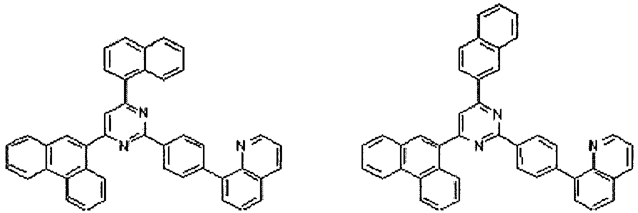

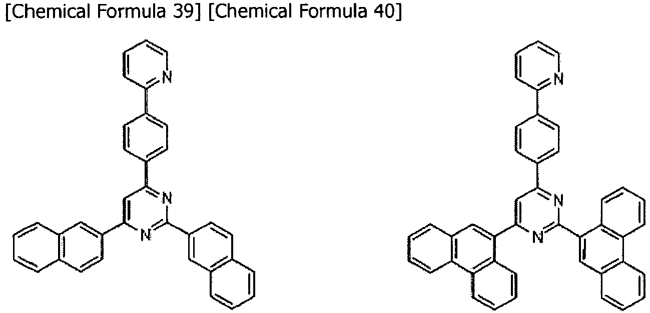

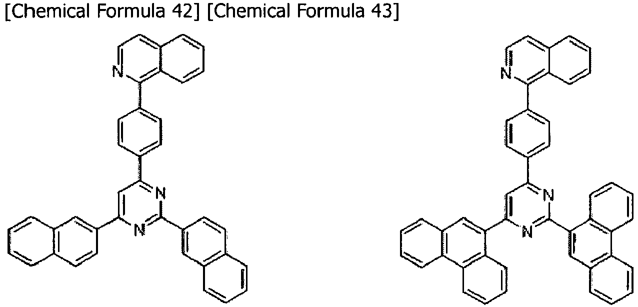

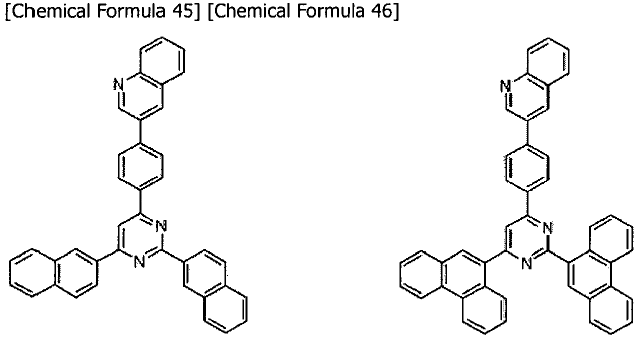

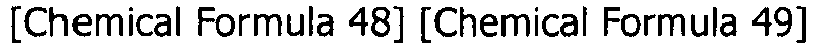

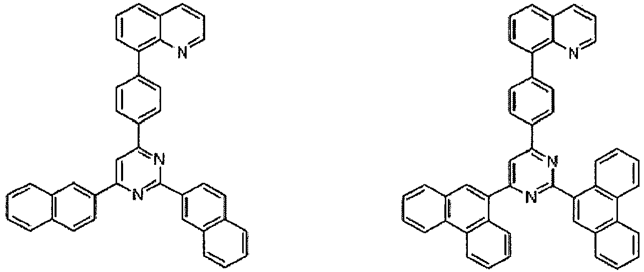

5. An organic photoelectric device comprising an anode, a cathode, and at least one organic thin layer interposed between the anode and cathode,
wherein at least one of the organic thin layer comprises the compound according to one of claims 1-4.

6. The organic photoelectric device of claim 5, wherein the organic thin layer comprises one selected from the group consisting of an emission layer, a hole transport layer (HTL), a hole injection layer (HIL), an electron injection layer (EIL), a hole blocking layer, and a combination thereof.

7. The organic photoelectric device of claim 5, wherein the compound is included in an electron transport layer (ETL) or an electron injection layer (EIL).

8. The organic photoelectric device of claim 5, wherein the compound is included in an emission layer.

9. The organic photoelectric device of claim 5,
wherein the compound is included in an emission layer as a phosphorescent or fluorescent host.

10. The organic photoelectric device of claim 5, wherein the compound is included in an emission layer as a fluorescent blue dopant.

11. The organic photoelectric device of claim 5, wherein the organic photoelectric device is selected from the group consisting of an organic light emitting diode, an organic solar cell, an organic transistor, an organic photo-conductor drum, and an organic memory device.

12. A display device comprising the organic photoelectric device according to claim 5.

## Patentansprüche

1. Verbindung für eine organische fotoelektrische Vorrichtung, die durch die folgende chemische Formel 1 dargestellt wird: 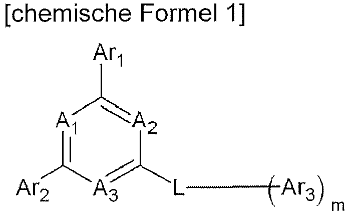 wobei in der vorstehenden chemischen Formel 1
A₁ bis A₃ gleich oder verschieden und unabhängig voneinander Kohlenstoff oder Sauerstoff bezeichnen, vorausgesetzt, dass zwei Komponenten von A₁ bis A₃ Stickstoff und eine Kohlenstoff ist;
Ar₁ und Ar₂ gleich oder verschieden sind und unabhängig voneinander ausgewählt sind aus der Gruppe bestehend aus Naphthyl, Anthracenyl, Phenanthrenyl, Pyrenyl, Perylenyl, Chrysenyl;
Ar₃ unsubstituiertes C₂- bis C₃₀-Heteroaryl ist;
L unsubstituiertes Phenylen, Naphthalen oder Anthracen ist, und
m eine ganze Zahl im Bereich von 1 bis 3 ist.

2. Verbindung nach Anspruch 1, wobei Ar₃ aus der Gruppe bestehend Imidazol, Thiazolyl, Oxazolyl, Oxadiazolyl, Triazolyl, Pyridinyl, Pyridazin, Chinolinyl, Isochinolinyl, Acridinyl, Imidazopyridinyl, Imidazopyrimidinyl ausgewählt ist.

3. Verbindung nach Anspruch 1, wobei L Phenylen ist.

4. Verbindung nach Anspruch 1, wobei die Verbindung eine durch die folgenden chemischen Formeln 3 bis 5, 9, 10, 12 bis 14, 18, 19, 21 bis 23, 27, 28, 30 bis 32, 36, 37, 39, 40, 42, 43, 45, 46, 48 und 49 dargestellte Verbindung oder eine Mischung davon aufweist:

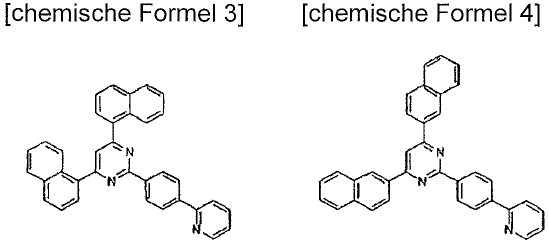

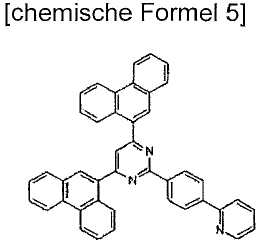

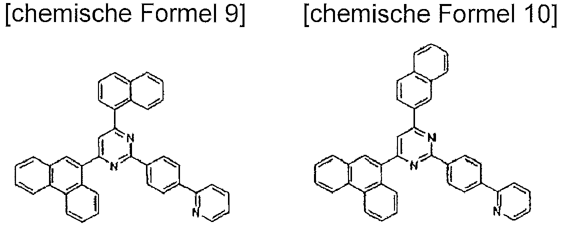

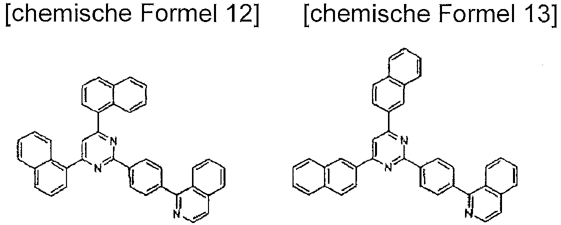

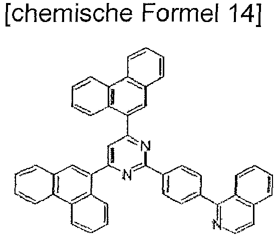

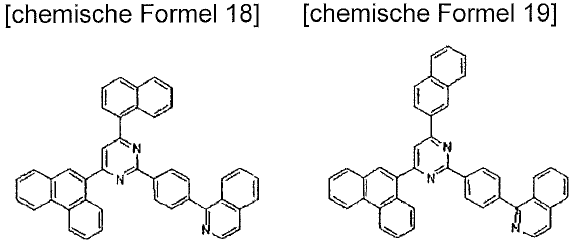

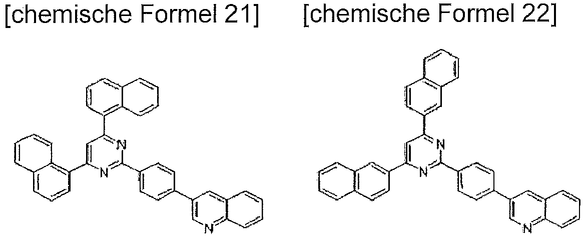

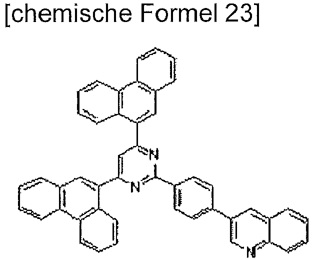

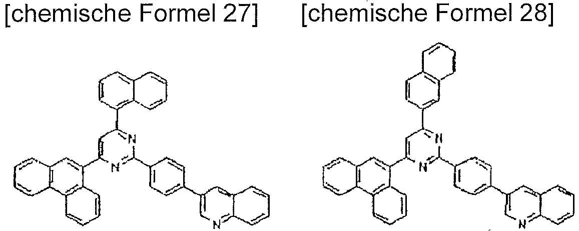

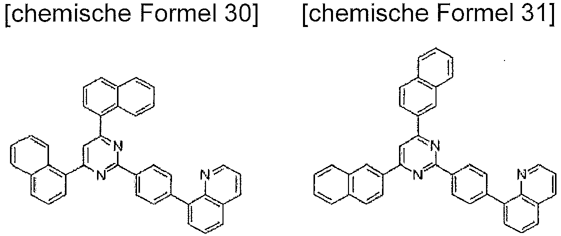

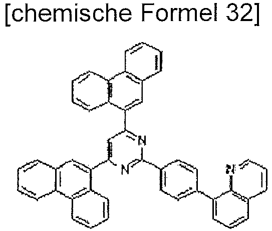

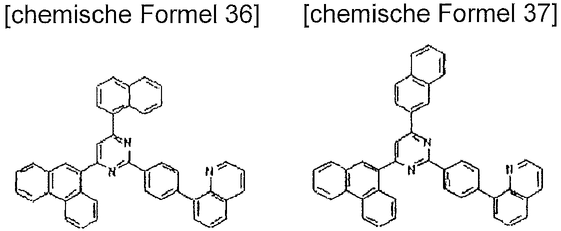

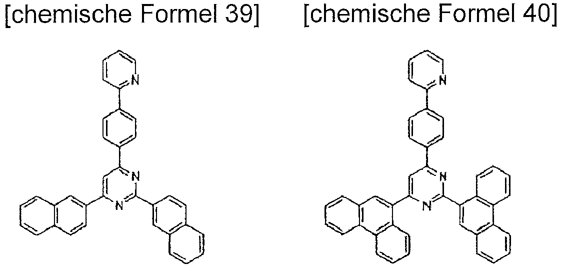

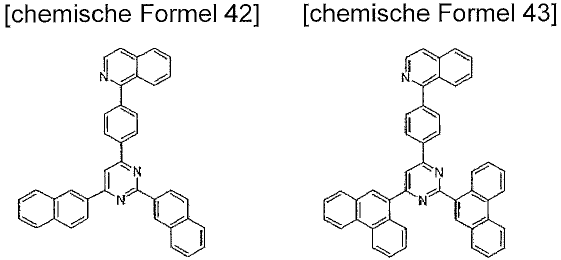

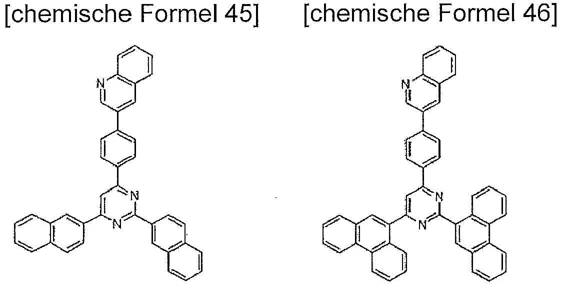

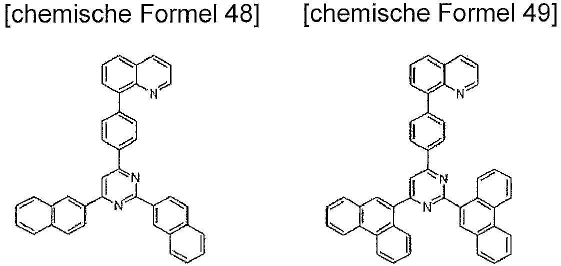

5. Organische fotoelektrische Vorrichtung mit einer Anode, einer Kathode und mindestens einer zwischen der Anode und der Kathode angeordneten organischen Dünnschicht, wobei mindestens eine Schichtlage der organischen Dünnschicht die Verbindung nach einem der Ansprüche 1 bis 4 aufweist.

6. Vorrichtung nach Anspruch 5, wobei die organische Dünnschicht mindestens eine Schichtlage aufweist, die ausgewählt ist aus der Gruppe bestehend aus einer Emissionsschicht, einer Lochtransportschicht (HTL), einer Lochinjektionsschicht (HIL), einer Elektroneninjektionsschicht (EIL), einer Lochblockierschicht und einer Kombination davon.

7. Vorrichtung nach Anspruch 5, wobei die Verbindung in einer Elektronentransportschicht (ETL) oder in einer Elektroneninjektionsschicht (EIL) angeordnet ist.

8. Vorrichtung nach Anspruch 5, wobei die Verbindung in einer Emissionsschicht angeordnet ist.

9. Vorrichtung nach Anspruch 5, wobei die Verbindung als ein phosphoreszierendes oder fluoreszierendes Hostmaterial in einer Emissionsschicht angeordnet ist.

10. Vorrichtung nach Anspruch 5, wobei die Verbindung als ein fluoreszierendes blaues Dotiermittel in einer Emissionsschicht enthalten ist.

11. Vorrichtung nach Anspruch 5, wobei die organische fotoelektrische Vorrichtung aus der Gruppe bestehend aus einer organischen Leuchtdiode, einer organischen Solarzelle, einem organischen Transistor, einer organischen Fotoleitertrommel und einer organischen Speichereinrichtung ausgewählt ist.

12. Displayvorrichtung mit der organischen fotoelektrischen Vorrichtung nach Anspruch 5.

## Revendications

1. Composé pour un dispositif photoélectrique organique représenté par la Formule Chimique 1 suivante : 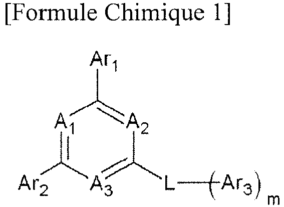 dans lequel dans la Formule Chimique 1 ci-dessus,
A₁ à A₃ sont identiques ou différents, et indépendamment un atome de carbone ou d'azote, à la condition que deux de A₁ à A₃ sont des atomes d'azote et un est un atome de carbone,
Ar₁ et Ar₂ sont identiques ou différents, et indépendamment sélectionnés dans le groupe constitué par des groupes naphthyle, anthracényle, phénanthrényle, pyrènyle, perylènyle, chrysènyle,
Ar₃ est un groupe hétéroaryle en C2 à C30 non substitué,
L est un groupe phénylène, naphtalène, ou anthracène non substitué, et
m est un nombre entier allant de 1 à 3.

2. Composé selon la revendication 1, dans lequel Ar₃ comprend un élément sélectionné dans le groupe constitué par des groupes imidazole, thiazolyle, oxazolyle, oxadiazolyle, triazolyle, pyridinyle, pyridazine, quinolinyle, isoquinolinyle, acridinyle, imidazopyridinyle, imidazopyrimidinyle.

3. Composé selon la revendication 1, dans lequel L est un groupe phénylène.

4. Composé selon la revendication 1, dans lequel le composé comprend un élément sélectionné dans les Formules Chimiques 3 à 5, 9, 10, 12 à 14, 18, 19, 21 à 23, 27, 28, 30 à 32, 36, 37, 39, 40, 42, 43, 45, 46, 48, et 49 suivantes, ou un mélange de celles-ci :

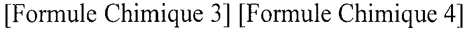

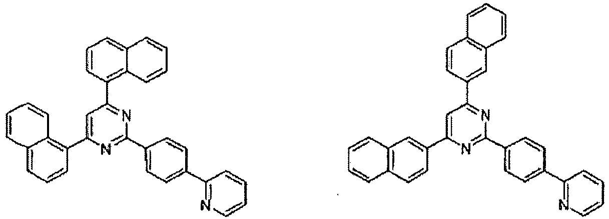

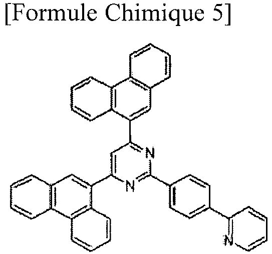

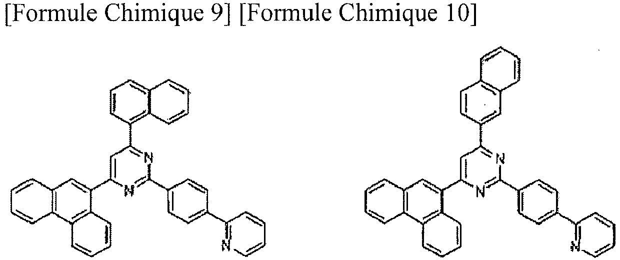

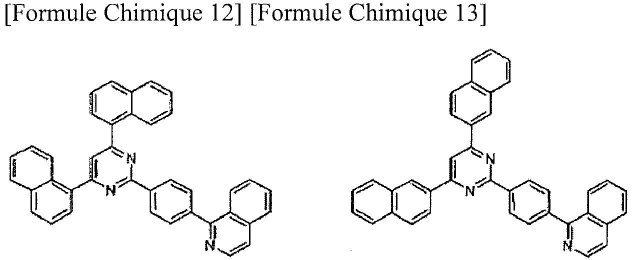

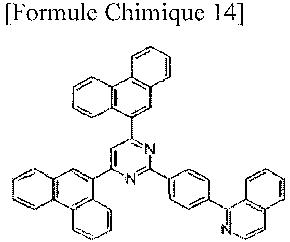

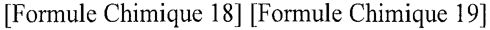

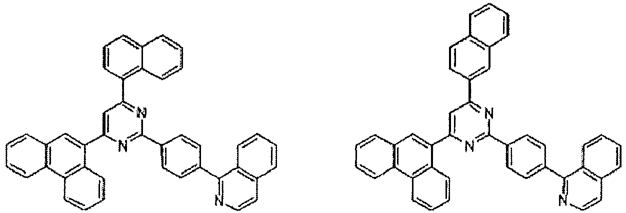

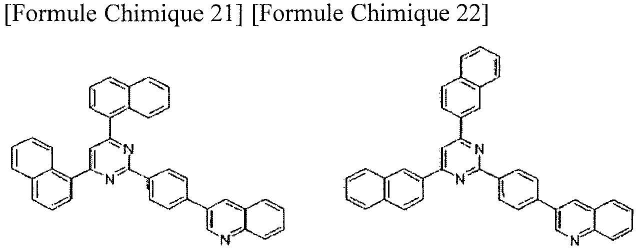

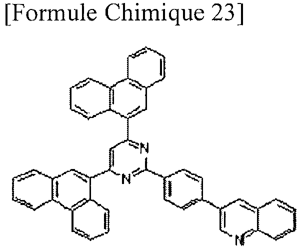

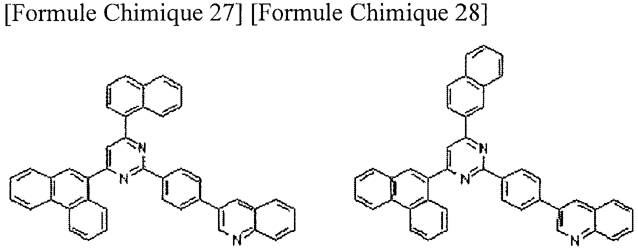

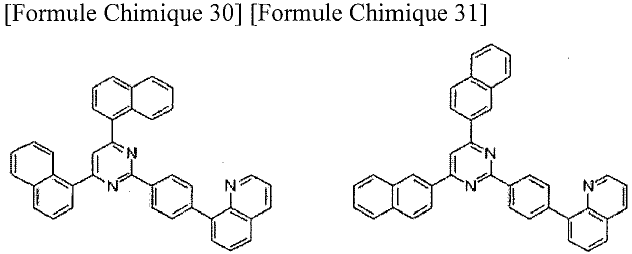

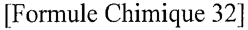

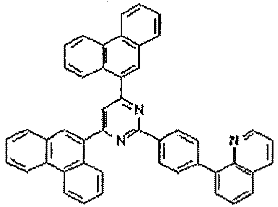

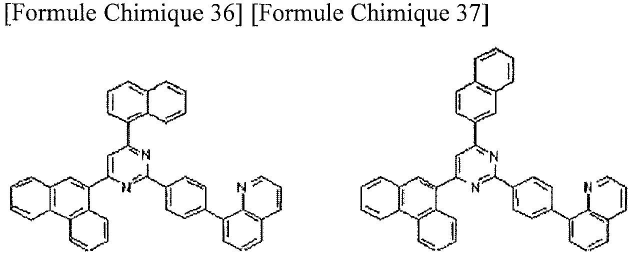

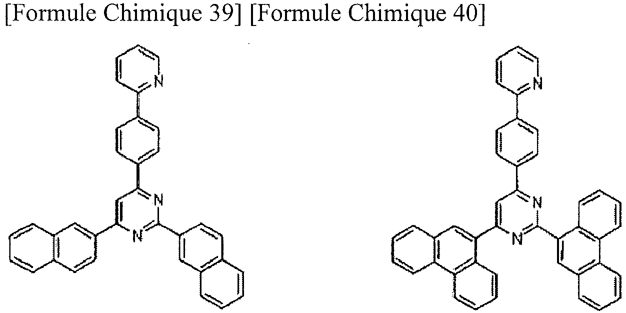

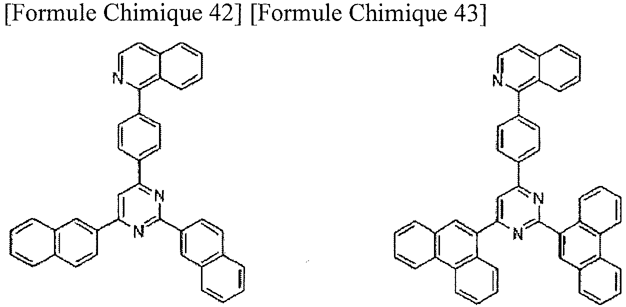

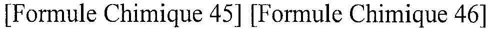

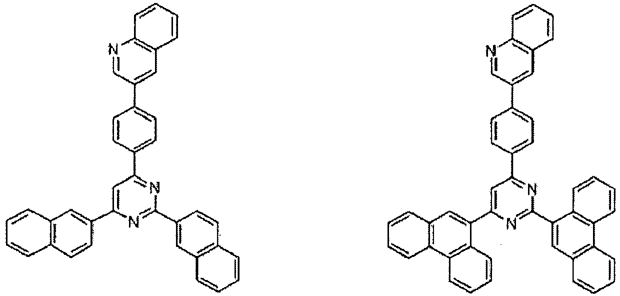

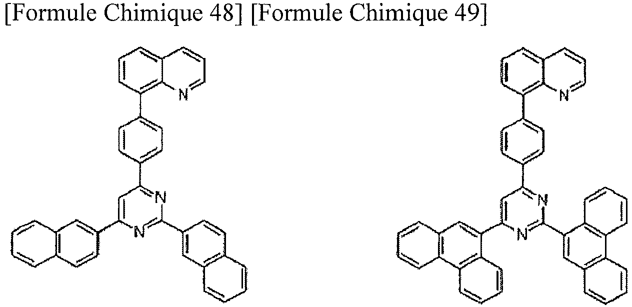

5. Dispositif photoélectrique organique comprenant une anode, une cathode, et au moins une couche mince organique interposée entre l'anode et la cathode,
dans lequel au moins une de la couche mince organique comprend le composé selon une des revendications 1-4.

6. Dispositif photoélectrique organique selon la revendication 5, dans lequel la couche mince organique comprend un élément sélectionné dans le groupe constitué par une couche d'émission, une couche de transport de trous (HTL), une couche d'injection de trous (HIL), une couche d'injection d'électrons (EIL), une couche de blocage de trous, et une combinaison de celles-ci.

7. Dispositif photoélectrique organique selon la revendication 5, dans lequel le composé est inclus dans une couche de transport d'électrons (ETL) ou une couche d'injection d'électrons (EIL).

8. Dispositif photoélectrique organique selon la revendication 5, dans lequel le composé est inclus dans une couche d'émission.

9. Dispositif photoélectrique organique selon la revendication 5, dans lequel le composé est inclus dans une couche d'émission comme un hôte phosphorescent ou fluorescent.

10. Dispositif photoélectrique organique selon la revendication 5, dans lequel le composé est inclus dans une couche d'émission comme un dopant bleu fluorescent.

11. Dispositif photoélectrique organique selon la revendication 5, dans lequel le dispositif photoélectrique organique est sélectionné dans le groupe constitué par une diode électroluminescente organique, une cellule photovoltaïque organique, un transistor organique, un tambour photoconducteur organique, et un dispositif de mémoire organique.

12. Dispositif d'affichage comprenant le dispositif photoélectrique organique selon la revendication 5.
